# FASCICULE DE BREVET EUROPEEN

(11) **EP 0 820 794 B1**
(45) Date de publication et mention de la délivrance du brevet: **08.10.2003**
(21) Numéro de dépôt: 97401374.0
(22) Date de dépôt: 17.06.1997
(51) Int. Cl.: B01D 3/00, C07C 41/42

(54) **Dispositif de réaction et de distillation et procédé d'éthérification**
Reaktions- und Destillationsvorrichtung sowie Verfahren zur Herstellung von Äthern
Reaction and distillation apparatus and process for the production of ethers

(30) Priorité: 26.07.1996 FR 9609553
(43) Date de publication de la demande: 28.01.1998
(73) Titulaire: Institut Français du Pétrole, 92852 Rueil-Malmaison Cedex (FR)
(72) Inventeur: Marion, Marie-Claire, 69100 Villeurbanne (FR); Thille, Christophe, 6001 Charleroi (BE); Forestiere, Alain, 69390 Vernaison (FR); Viltard, Jean-Charles, 26000 Valence (FR)

(56) Documents cités:
- EP-A- 0 537 636
- WO-A-93/20032
- US-A- 4 503 265
- US-A- 5 364 975

## Description

La présente invention concerne un dispositif de réaction et de distillation et un procédé de fabrication d'un éther alkylique tertiaire par réaction d'au moins une oléfine avec un monoalcool aliphatique, généralement en excès.

Il est connu de préparer des éthers alkyliques tertiaires en faisant réagir une iso-oléfine généralement contenue dans une fraction d'hydrocarbures avec un alcool aliphatique, utilisé en général en excès, en présence d'un catalyseur acide, par exemple l'acide sulfurique, l'acide fluorhydrique, le chlorure d'aluminium ou le fluorure de bore, ou en présence de matières carbonées contenant des groupes sulfoniques (-SO3H), par exemple des charbons sulfonés, des résines phénol-formaldéhydes sulfonées, des polymères coumarones-indène sulfonés ou, de préférence, des résines de copolymères styrène-divinylbenzène sulfonées ou d'autres composés, notamment des composés minéraux, comportant des groupes sulfoniques (par exemple des polysiloxanes sulfonés).

On sait depuis longtemps que la réaction entre un monoalcool aliphatique et une oléfine tertiaire est équilibrée et qu'il est difficile d'obtenir des taux de conversion des iso-oléfines avec une pureté et un rendement élevés. Les procédés classiques tels que décrits par exemple dans Hydrocarbon Technology International, Autumn 95, p. 21-27 comprennent un ou plusieurs réacteur(s) d'éthérification en éthers alkyliques tertiaires suivi(s) par au moins une zone de fractionnement généralement de distillation dont le produit de fond est de l'éther contenant des quantités aussi faibles que possible de monoalcool(s).

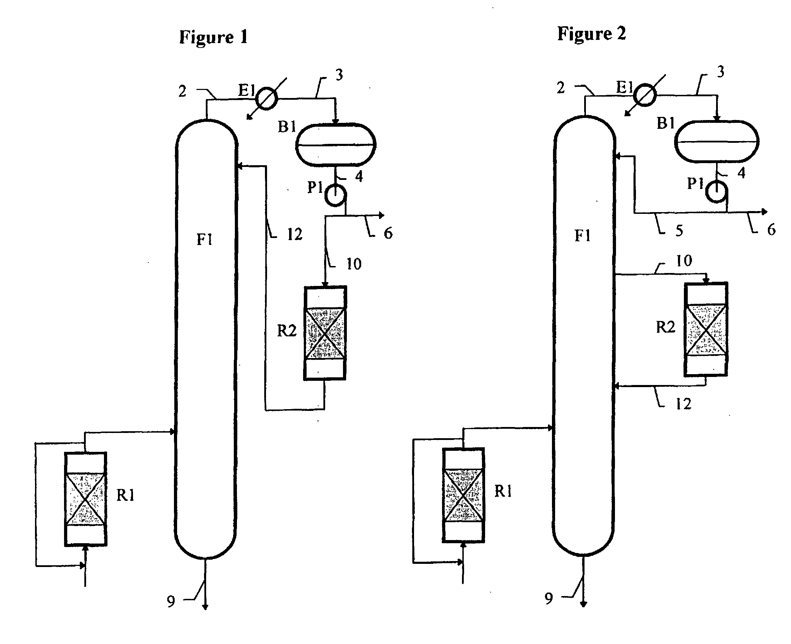

C'est pourquoi l'art antérieur a préconisé, en vue d'améliorer les performances de cette synthèse, d'ajouter au réacteur principal une section réactionnelle complémentaire comme cela est par exemple décrit dans le brevet au nom de la demanderesse US-A-5.364.975. Dans ce brevet la section réactionnelle complémentaire est selon une forme préférée de réalisation incluse sur le reflux de la section de fractionnement. Il a encore été proposé, par exemple dans le brevet US-A-4.503.265 ou dans les demandes de brevet WO-A-93/19.031 et WO-A-93/19.032, de prélever un produit sur un plateau intermédiaire de la section de fractionnement, d'envoyer ce produit dans une section réactionnelle complémentaire et de renvoyer le produit issu de cette section réactionnelle complémentaire dans la section de fractionnement à un niveau inférieur au niveau de prélèvement. Cette réalisation présente l'inconvénient de perturber le bon fonctionnement en distillation de la section de fractionnement. Il a également été décrit par exemple dans Hydrocarbon processing March 1995 page 114 un procédé dénommé Ethermax de la société UOP dans lequel l'effluent de la section principale d'éthérification est envoyé dans une zone de distillation-réaction. Ce procédé présente l'inconvénient, comme cela sera montré ci-après dans un exemple comparatif de nécessiter une colonne de très grande hauteur si on veut améliorer très sensiblement les performances globales. Ces procédés classiques de fabrication d'éthers alkyliques tertiaires seront décrits ci-après, en liaison avec les figures 1, 2 et 3.

L'un des objets de l'invention est de palier aux principaux inconvénients des procédés décrits dans l'art antérieur et de proposer plusieurs modes de réalisation de synthèse d'éthers permettant de maximiser la conversion globale des iso-oléfines contenues dans des coupes d'hydrocarbures.

Dans la section réactionnelle principale représentée par le réacteur R1 sur les figures 1 à 9, on introduit la charge constituée d'un mélange d'hydrocarbures en C4, C5, C6 ou C7 comprenant des iso-oléfines et au moins un monoalcool aliphatique habituellement utilisé en excès. Les réactifs mélangés sont mis en contact avec un catalyseur acide.

Le produit issu de cette section réactionnelle R1 est envoyé dans une zone de distillation représentée par la colonne F1 sur les figures 1 et 2 et par la colonne F2 sur la figure 3. Dans cette colonne, il est distillé, pour produire en fond, par la ligne 9, un éther alkylique tertiaire contenant des quantités aussi faibles que possible de monoalcool(s), et en tête, par la ligne 2, un mélange d'hydrocarbures réactifs et non réactifs et de monoalcool(s) aliphatique(s) entraîné(s) par azéotropie. Cet effluent sortant par la ligne 2 est condensé dans le condenseur E1 et recueilli par la ligne 3 dans le ballon B1, d'où il est repris, par la ligne 4, par la pompe P1.

Dans le cas schématisé sur la figure 1 (brevet US-A-5.364.975), une partie de l'effluent sortant de la pompe P1 est envoyée par la ligne 10 vers un réacteur R2, dit réacteur de finition, dont l'effluent est envoyé par la ligne 12 dans la colonne F1 à titre de reflux et le reste de l'effluent sort comme distillat par la ligne 6.

Dans le mode de réalisation tel que schématisé sur la figure 2 (brevet US-A-4.503.265), on prélève un produit sur un plateau intermédiaire de la colonne de distillation entre le point d'introduction de l'effluent du réacteur R1 d'ethérification et le sommet de cette colonne et on envoie ce produit par la ligne 10 dans un réacteur R2, dit réacteur de finition, dont l'effluent est envoyé par la ligne 12 dans la colonne F1 à un niveau inférieur au niveau du point de prélèvement. Dans le cas schématisé sur la figure 2 une partie de l'effluent sortant de la pompe P1 est envoyé par la ligne 5 dans la colonne F1 à titre de reflux et le reste de l'effluent sort comme distillat par la ligne 6.

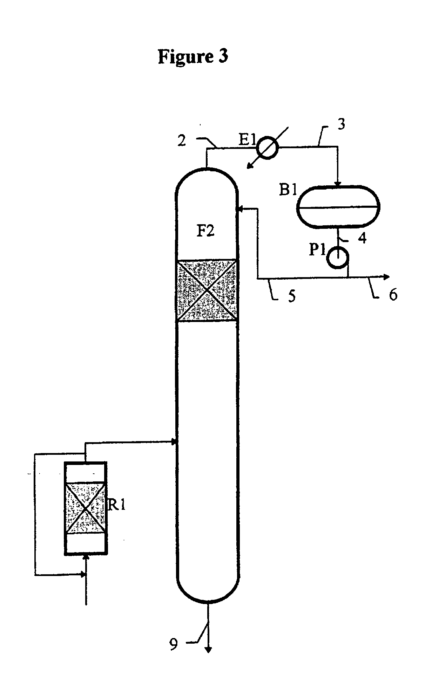

Dans le mode de réalisation tel que schématisé sur la figure 3 (Hydrocarbon processing March 1995 p. 114), l'effluent de la section réactionnelle R1 est envoyé dans une colonne de distillation-réaction en dessous du premier lit de catalyseur et une partie de l'effluent sortant de la pompe P1 est envoyé par la ligne 5 dans la colonne F1 à titre de reflux et le reste de l'effluent sort comme distillat par la ligne 6.

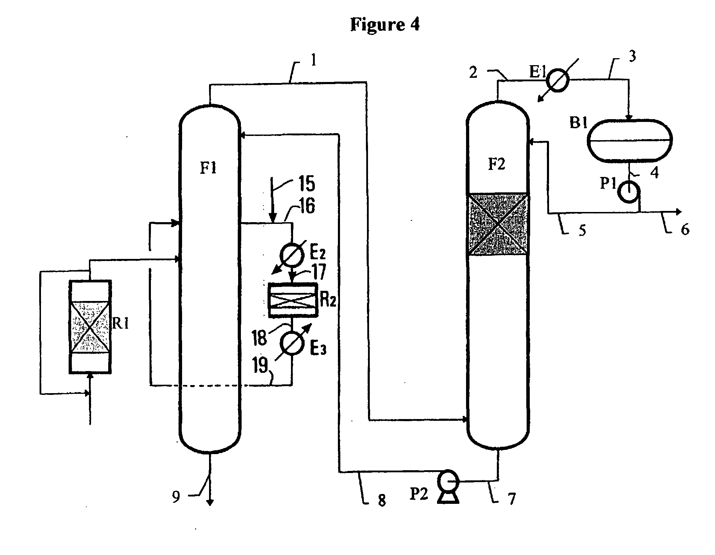

Cependant, un tel procédé nécessite dans le cas schématisé sur la figure 3 un grand nombre de zones réactionnelles dans la colonne de distillation-réaction F2 pour obtenir des conversions élevées en iso-oléfines et par conséquent, une hauteur de colonne imposante. En effet, l'obtention de conversions élevées nécessitent une colonne de distillation-réaction contenant de nombreuses zones réactionnelles. Sachant qu'une seule de ces zones occupe environ 3 mètres de hauteur dans la colonne de distillation-réaction, la hauteur finale de cette colonne devient très vite limitative. Il serait donc intéressant de pouvoir bénéficier d'une conversion en iso-oléfines aussi élevée tout en gardant un nombre limité de zones réactionnelles dans la colonne ou/et limiter la hauteur de la colonne. C'est l'un des objectifs que se fixe l'invention, telle quelle sera décrite ci-après, en liaison avec les figures 4,5,6,7 et 8.

La présente invention concerne un dispositif de distillation et de réaction pour la fabrication d'un éther alkylique tertiaire par réaction d'au moins une oléfine avec monoalcool aliphatique, comprenant :
- un réacteur comportant un moyen d'alimentation,
- une section de distillation-réaction comprenant
   - une colonne de distillation,
   - une colonne de distillation-réaction, comprenant un lit de catalyseur,
   - un réacteur complémentaire, comportant un lit de catalyseur, le réacteur complémentaire comprend un conduit d'introduction et un conduit d'évacuation,
- un moyen de circulation d'un effluent dudit réacteur à ladite colonne de distillation,
- un moyen de circulation d'un effluent de la tête de ladite colonne de distillation dans ladite colonne de distillation-réaction,
- un moyen de prélèvement de produit en fond de la colonne de distillation, ledit réacteur complémentaire est agencé selon l'une des dispositions suivantes :
   a) ledit conduit d'introduction et ledit conduit d'évacuation sont reliés à ladite colonne de distillation, et un moyen de circulation conduit un effluent du fond de ladite colonne de distillation-réaction à la tête de ladite colonne de distillation,
   b) ledit conduit d'introduction et ledit conduit d'évacuation sont reliés à ladite colonne de distillation-réaction, et un moyen de circulation conduit un effluent du fond de ladite colonne de distillation-réaction à la tête de ladite colonne de distillation,
   c) ledit conduit d'introduction est relié au fond de ladite colonne de distillation-réaction et ledit conduit d'évacuation est relié à la tête de ladite colonne de distillation.

Dans le dispositif selon l'invention, la colonne de distillation-réaction peut comporter au moins un dispositif de reflux. Le dispositif de reflux comprend au moins un moyen de prélèvement situé en tête de ladite colonne de distillation-réaction, alimentant au moins une zone de condensation, au moins un moyen de circulation d'une partie d'effluent de ladite zone de condensation vers la tête de ladite colonne de distillation-réaction et au moins un moyen de prélèvement d'une autre partie d'effluent de la zone de condensation.

Dans le dispositif selon la disposition a), le conduit d'introduction et le conduit d'évacuation du réacteur complémentaire peuvent être disposés à un même niveau de ladite colonne de distillation, le niveau pouvant être situé entre l'alimentation de ladite colonne de distillation et la tête de ladite colonne de distillation.

Dans le dispositif selon la disposition b), le conduit d'introduction et le conduit d'évacuation sont disposés à un même niveau de ladite colonne de distillation-réaction, le niveau pouvant être situé entre le fond de ladite colonne de distillation-réaction et le bas du lit de catalyseur situé le plus bas dans ladite colonne de distillation-réaction.

Le conduit d'introduction et/ou le conduit d'évacuation du réacteur complémentaire peut comprendre au moins un moyen de régulation de la température.

Le réacteur complémentaire peut comprendre au moins un moyen d'alimentation de réactif.

L'invention concerne également l'utilisation du dispositif selon l'invention pour préparer au moins un éther alkylique tertiaire par réaction d'au moins un monoalcool aliphatique et d'au moins une oléfine, dans laquelle:
a) on introduit dans ledit réacteur au moins une oléfine et au moins un alcool aliphatique,
b) on prélève en fond de la colonne de distillation de l'éther alkylique tertiaire,
c) on prélève en tête de la colonne de distillation-réaction un effluent comprenant des hydrocarbures n'ayant pas réagi et de l'alcool aliphatique.

On peut également introduire de l'alcool aliphatique supplémentaire dans le réacteur complémentaire.

Finalement, dans le procédé d'éthérification , la présence du réacteur complémentaire, permet avantageusement de réguler au mieux deux paramètres important que sont la température et l'ajout d'alcool dans ladite section, ce qui favorise l'optimisation de la conversion ou du rendement en éther(s).

Le dispositif selon l'invention tel que représenté sur les figures 4, 5, 6, 7 et 8 comprend une section de fractionnement F1. L'effluent de fond de la colonne de distillation-réaction F2 est utilisé comme reflux de la colonne de fractionnement F1, auquel cas, en présence du réacteur de finition R2, ledit réacteur est alimenté par un soutirage liquide issu d'un niveau de prélèvement de la colonne de distillation-réaction F2, l'effluent dudit réacteur R2 étant ensuite réinjecté légèrement au-dessous dudit niveau dans la colonne F2 ; ou bien l'effluent de fond de la colonne de distillation-réaction F2 est utilisé comme charge du réacteur de finition R2 dont le produit sert ensuite de reflux de la colonne de fractionnement F1 ; ou bien ledit réacteur est alimenté par un soutirage liquide issu de la colonne de distillation F1, l'effluent dudit réacteur R2 étant ensuite réinjecté légèrement au-dessous dudit niveau dans la colonne F1.

Le procédé selon l'invention est plus particulièrement un procédé de fabrication d'éther alkylique tertiaire à partir d'isobutène et d'éthanol ou d'autres monoalcools aliphatiques tels que le méthanol, le propanol ou l'isopropanol, ainsi que la fabrication d'autres éthers alkyliques tertiaires à partir d'iso-oléfines en C4, C5, C6 et C7 et de monoalcools aliphatiques de C1 à C4, en particulier le méthanol, l'éthanol, le propanol et l'isopropanol. Ainsi il concerne plus particulièrement la fabrication du méthyl tertio-butyl éther (MTBE), de l'éthyl tertio-butyl éther (ETBE) et du méthyl tertio-amyl éther (TAME). Dans tous les cas, l'alcool (les alcools) est (sont) généralement en excès par rapport à l'oléfine (aux oléfines).

La charge utilisée pour préparer l'éther alkylique tertiaire selon le procédé de l'invention contient en général au moins une iso-oléfine capable de réagir avec le monoalcool aliphatique dans la réaction d'ethérification donnant naissance à l'éther alkylique tertiaire.

Le plus souvent, mais ce n'est pas absolument indispensable, les charges traitées sont des coupes provenant de craquage catalytique et de craquage à la vapeur après fractionnement. Suivant le fractionnement, ces charges pourront contenir de faibles proportions d'hydrocarbures ayant un nombre d'atomes de carbone par molécule inférieur ou supérieur à celui des iso-oléfines à traiter.

Après réaction dans la section réactionnelle R1, menée dans des conditions classiques (généralement dans les conditions opératoires suivantes : en phase liquide ou mixte, à une pression de 0,2 à 3 MPa, de préférence de 0,5 à 2 MPa, à une température de 30 à 150°C, de préférence de 40 à 100°C), l'effluent sortant du réacteur R1 contient en général de l'éther alkylique tertiaire, les hydrocarbures non-réactifs contenus dans la charge, les hydrocarbures non-convertis et l'excès de monoalcool aliphatique. Cet effluent est envoyé dans la section de fractionnement, où il est distillé en général sous pression absolue de 0,3 à 1MPa et à une température de fond de 80 à 160°C.

L'effluent de tête sortant de la section de fractionnement F1 par la ligne 1 contient les hydrocarbures non-réactifs de la charge, les hydrocarbures non-convertis ainsi que l'excès de monoalcool aliphatique. Cet effluent est ensuite injecté dans la colonne de distillation-réaction F2. Le produit de fond de cette colonne de distillation-réaction est utilisé comme reflux de la colonne de fractionnement F1 et comprend des hydrocarbures non-réactifs, des hydrocarbures non-convertis dans la première section réactionnelle R1 et l'excès de monoalcool aliphatique et l'éther. L'effluent de fond sortant de la section de distillation-réaction F2 par la ligne 7 contient les hydrocarbures non-réactifs de la charge, les hydrocarbures non-convertis ainsi que du monoalcool aliphatique et de l'éther.

L'effluent soutiré en fond par la ligne 9 comprend un éther alkylique tertiaire contenant des quantités aussi faibles que possible de mono alcool(s).
On récupère par la ligne 2 en tête de colonne de fractionnement F2, un mélange d'hydrocarbures réactifs et non réactifs et de monoalcool(s) aliphatiques entraînés par azéotropie.

Le catalyseur acide utilisé dans le réacteur R1, ainsi que dans la zone de distillation-réaction et dans le réacteur R2, est généralement choisi parmi tous les catalyseurs connus de l'homme du métier pour réaliser la réaction considérée. Il peut être choisi par exemple parmi l'acide sulfurique, l'acide fluorhydrique, le chlorure d'aluminium, le fluorure de bore, des matières carbonées, sulfonées, telles que des carbones sulfonés, des résines phénol-formaldéhyde sulfonées, des polymères coumarone-indène sulfonés ou, de préférence, des résines copolymères-styrène-divinylbenzène sulfonées. On peut aussi utiliser un catalyseur zéolitique. On peut encore utiliser d'autres composés, notamment des composés minéraux, comportant des groupes sulfoniques (par exemple des polysiloxanes sulfonés).

La figure 4 représente schématiquement un mode de réalisation selon l'invention du procédé de préparation d'éther alkylique tertiaire dans lequel l'effluent de la section réactionnelle d'ethérification R1 est envoyé dans une colonne de fractionnement F1 dans laquelle il est distillé, pour produire en fond, par la ligne 9, un éther alkylique tertiaire contenant des quantités aussi faibles que possible de monoalcool(s), et en tête, par la ligne 1, un mélange d'hydrocarbures et de monoalcool aliphatique entraîné par azéotropie. Ce produit de tête est envoyé dans une colonne de distillation-réaction F2. Le produit de fond de cette colonne de distillation-réaction F2 sortant par la ligne 7 est ensuite envoyé par la pompe P2 et la ligne 8 comme reflux de la colonne de fractionnement F1. Dans le mode de réalisation du procédé comprenant un réacteur de finition alimenté par un soutirage liquide (ligne 16) issu de la colonne de distillation F1, l'effluent de ce réacteur étant réinjecté sensiblement légèrement en dessous du niveau de prélèvement dans la colonne F1 (ligne 19). La charge de ce réacteur de finition R2 est mélangée avec une quantité complémentaire d'au moins un monoalcool aliphatique introduite par la ligne 15. La température de ce mélange est ensuite ajustée dans l'échangeur de chaleur E2. Le produit issu de cet échangeur est ensuite envoyé dans le réacteur R2 par la ligne 17. L'effluent de ce réacteur est introduit dans l'échangeur de chaleur E3 par la ligne 18 pour que sa température soit ajustée. L'effluent de cet échangeur E3 est injecté dans la colonne de distillation-réaction par la ligne 19. Le soutirage d'un produit liquide est généralement effectué au-dessus du point d'alimentation de la zone de distillation.

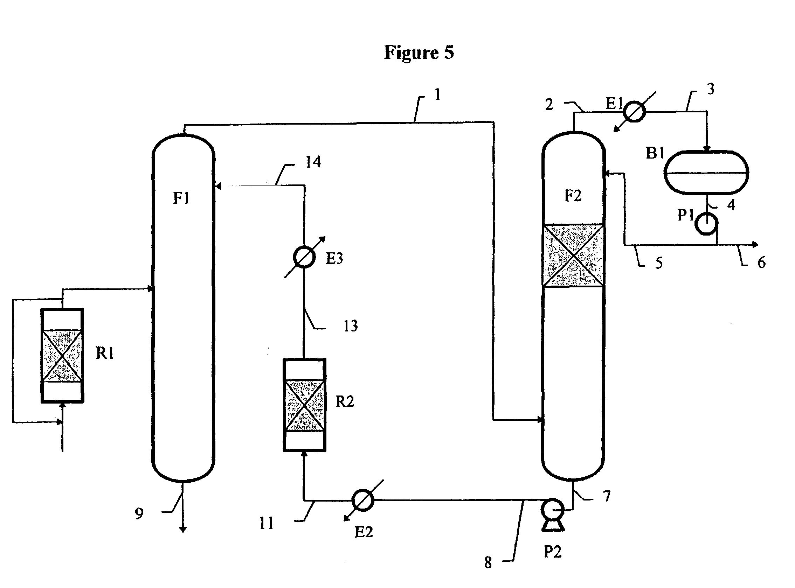

La figure 5 représente schématiquement un autre mode de réalisation selon l'invention du procédé de préparation d'éther alkylique tertiaire comprenant un réacteur de finition sur le reflux de la colonne de fractionnement F1 et dont la température de la charge du réacteur de finition R2 est ajustée par l'intermédiaire de l'échangeur de chaleur E2 et la température de l'effluent de ce même réacteur R2 est également ajustée par l'intermédiaire de l'échangeur de chaleur E3. Ainsi le produit sortant de la pompe P2 est envoyé dans l'échangeur de chaleur E2 par la ligne 8 et l'effluent sortant de cet échangeur de chaleur E2 par la ligne 11 est utilisé comme charge du réacteur de finition R2 et l'effluent sortant du réacteur R2 par la ligne 13 est ensuite envoyé dans l'échangeur de chaleur E3. L'effluent sortant de cet échangeur de chaleur E3 par la ligne 14 est ensuite utilisé comme reflux de la colonne de fractionnement F1. Les autres éléments schématisés sur cette figure sont identiques à ceux décrits en liaison avec les figures précédentes.

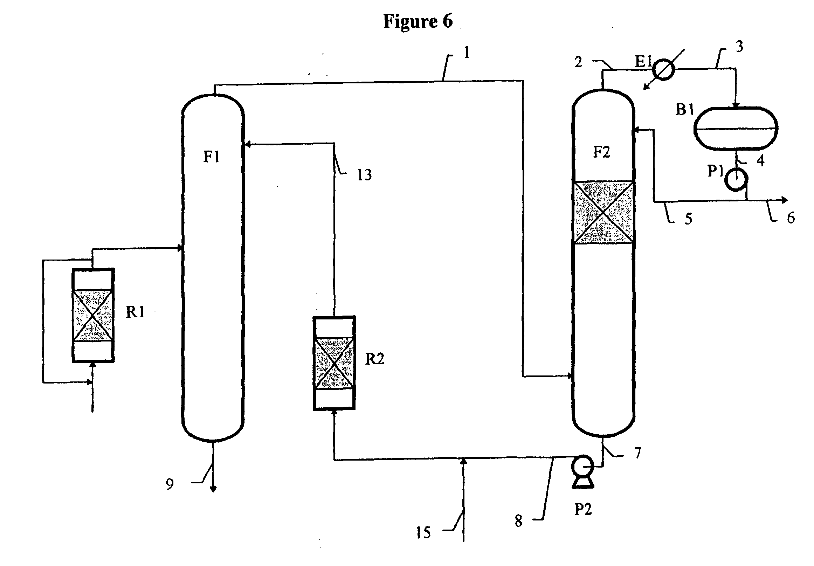

La figure 6 représente schématiquement un autre mode de réalisation selon l'invention du procédé dans lequel on injecte par la ligne 15, en amont du réacteur de finition R2, une quantité complémentaire d'au moins un monoalcool aliphatique, qui peut être le même alcool que l'alcool mis en jeu dans le réacteur principal d'éthérification, ou un monoalcool différent, par exemple choisi parmi le méthanol, l'éthanol, le propanol et l'isopropanol. Ainsi le produit sortant de la pompe P2, par la ligne 8, est mélangé avec une quantité d'au moins un monoalcool aliphatique, issu de la ligne 15, et le mélange est envoyé dans le réacteur de finition R2. L'effluent sortant du réacteur R2 par la ligne 13 est ensuite utilisé comme reflux de la colonne de fractionnement F1. Les autres éléments schématisés sur cette figure sont identiques à ceux décrits en liaison avec les figures précédentes.

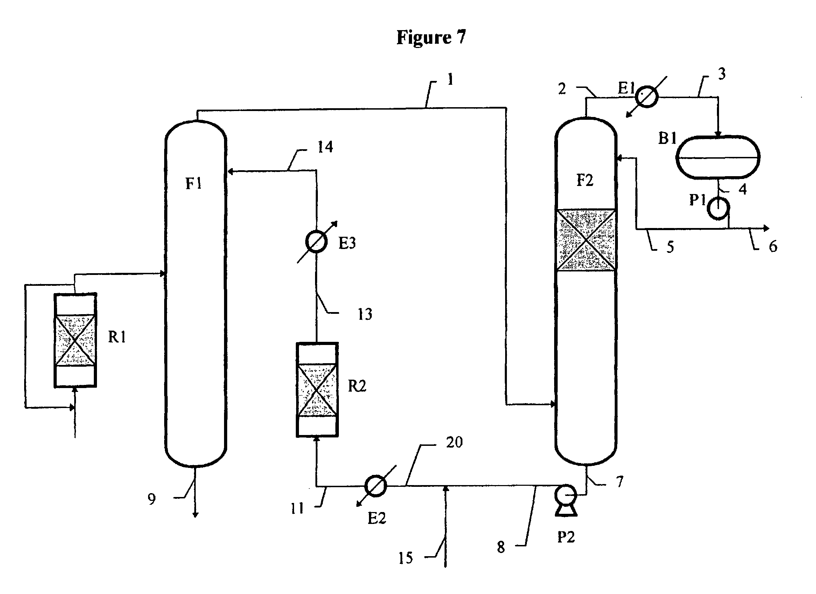

La figure 7 représente schématiquement un autre mode de réalisation selon l'invention du procédé qui regroupe les modes de réalisation des figures 5 et 6 par le fait que l'on ajoute une quantité complémentaire d'au moins un alcool aliphatique en amont du réacteur de finition R2 par la ligne 15 et que la température de la charge et de l'effluent de ce même réacteur est ajustée. Ainsi selon cette réalisation le produit sortant de la pompe P2 par la ligne 8 est mélangé avec une quantité d'au moins un monoalcool aliphatique, issu de la ligne 15. Ce mélange est envoyé, par la ligne 20, dans l'échangeur de chaleur E2. L'effluent sortant de cet échangeur de chaleur E2 par la ligne 11 est utilisé comme charge du réacteur de finition R2. L'effluent sortant du réacteur R2 par la ligne 13 est ensuite envoyé dans l'échangeur de chaleur E3. L'effluent sortant de cet échangeur de chaleur E3 par la ligne 14 est ensuite utilisé comme reflux de la colonne de fractionnement F1. Les autres éléments schématisés sur cette figure sont identiques à ceux décrits en liaison avec les figures précédentes.

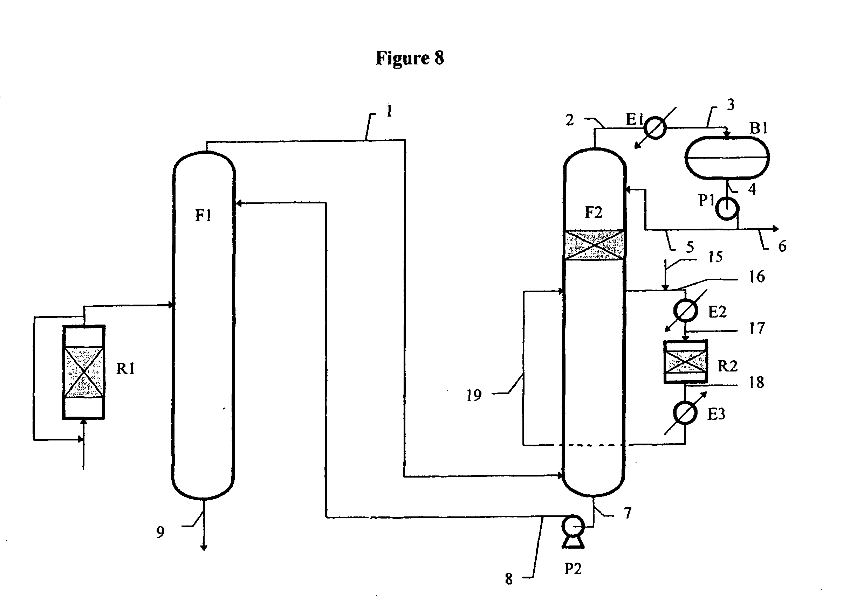

La figure 8 représente un autre mode de réalisation du procédé comprenant un réacteur de finition alimenté par un soutirage liquide issu de la colonne de distillation-réaction F2, l'effluent de ce réacteur étant réinjecté sensiblement légèrement au-dessous du niveau de prélèvement de la colonne F2. La charge de ce réacteur de finition R2 (ligne 16) est mélangée avec une quantité complémentaire d'au moins un monoalcool aliphatique introduite par la ligne 15. La température de ce mélange est ensuite ajustée dans l'échangeur de chaleur E2. Le produit issu de cet échangeur est ensuite envoyé dans le réacteur R2 par la ligne 17. L'effluent de ce réacteur est introduit dans l'échangeur de chaleur E3 par la ligne 18 pour que sa température soit ajustée. L'effluent de cet échangeur E3 est injecté dans la colonne de distillation-réaction par la ligne 19.

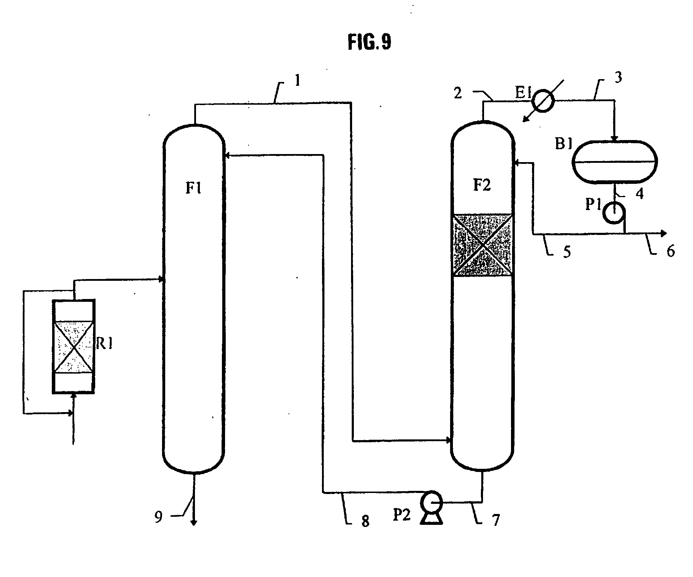

La figure 9 représente schématiquement un mode de réalisation qui ne correspond pas au procédé de préparation d'éther alkylique tertiaire selon l'invention. Ici l'effluent de la section réactionnelle d'ethérification R1 est envoyé dans une colonne de fractionnement F1 dans laquelle il est distillé, pour produire en fond, par la ligne 9, un éther alkylique tertiaire contenant des quantités aussi faibles que possible de monoalcool(s), et en tête, par la ligne 1, un mélange d'hydrocarbures et de monoalcool aliphatique entraîné par azéotropie. Ce produit de tête est envoyé dans une colonne de distillation-réaction F2. Le produit de fond de cette colonne de distillation-réaction F2 sortant par la ligne 7 est ensuite envoyé par la pompe P2 et la ligne 8 comme reflux de la colonne de fractionnement F1.

Les exemples suivants illustrent l'invention. Les exemples 1, et 3 sont comparatifs.

### Exemples 1 à 8

On a effectué la préparation de l'éthyl tertio-butyl éther (ETBE), d'une part, selon le schéma classique (schémas 1, 2 et 3, en liaison respectivement avec les figures 1, 2 et 3), et d'autre part selon les modes de réalisation décrits dans l'invention (schémas 4, 5, 6, 7, et 9 en liaison respectivement avec les figures 4, 5, 6, 7, 8 et 9).

Dans le tableau suivant sont indiquées la composition de la charge et la conversion d'isobutène dans la section réactionnelle principale R1 et les conditions opératoires mises en oeuvre pour chaque schéma, dans le réacteur de finition lorsqu'il est présent, ainsi que la conversion globale, la quantité d'éthanol obtenue en fond de colonne de fractionnement F1 et en tête de colonne de distillation-réaction F2.

Conditions opératoires dans le réacteur de finition :

| | |
|---|---|
| Catalyseur | résine acide (copolymère styrène-divinylbenzène sulfoné) |
| Type de réacteur | lit fixe |
| Phase | liquide |
| Pression | 7,8.10⁵Pa |

| Caractéristiques | Fig. 1 | Fig. 2 | Fig. 3 | Fig. 4 | Fig. 5 | Fig. 6 | Fig. 7 | Fig. 8 | Fig. 9 |
|---|---|---|---|---|---|---|---|---|---|
| Teneur en isobutène dans la charge entrant dans la section réactionnelle R1 (% poids) | 22,9 | 22,9 | 22,9 | 22,9 | 22,9 | 22,9 | 22,9 | 22,9 | 22,9 |
| Conversion de l'isobutène dans la section réactionnelle R1 (%) | 92,3 | 92,3 | 92,3 | 92,3 | 92,3 | 92,3 | 92,3 | 92,3 | 92,3 |
| Nombre de zones réactionnelles dans la colonne de distillation-réaction F2 | 0 | 0 | 9 | 5 | 5 | 5 | 5 | 5 | 5 |
| Teneur en éthanol dans le raffinat de la colonne de distillation-réaction F2 (% poids) | 1,5 | 1,4 | 0,47 | 1,0 | 0,6 | 0,9 | 1,0 | 1,0 | 0,47 |
| Teneur en éthanol dans le fond de la colonne de fractionnement (F1 ou F2 fig. 3) (% poids) | 3,5 | 3,5 | 3,5 | 3,5 | 3,5 | 3,5 | 3,5 | 3,5 | 3,5 |
| Rapport molaire éthanol/isobutène à l'entrée de la section réactionnelle R1 | 1,05 | 1,05 | 1,05 | 1,05 | 1,05 | 1,05 | 1,05 | 1,05 | 1,05 |
| Rapport molaire éthanol/isobutène à l'entrée de la colonne de fractionnement F1 | 1,86 | 1,86 | 1,86 | 1,86 | 1,86 | 1,86 | 1,86 | 1,86 | 1,86 |
| Rapport molaire éthanol/isobutène à l'entrée du réacteur de finition R2 | 0,8 | 2,0 | ** | 3,7 | 1,4 | 4,0 | 3,6 | 3,6 | ** |
| Rapport molaire éthanol/isobutène sur l'ensemble de l'unité réactionnelle | 1,05 | 1,05 | 1,05 | 1,08 | 1,05 | 1,08 | 1,08 | 1,08 | 1,05 |
| Température à l'entrée du réacteur de finition R2 (°C) | 57,2 | 68,6 | ** | 55 | 55 | 72,5 | 54,5 | 54,5 | ** |
| Température à la sortie du réacteur de finition R2 (°C) | 59,5 | 69,8 | ** | 56,3 | 56,6 | 73,5 | 56,3 | 56,2 | ** |
| Conversion globale d'isobutène (%) | 94,50 | 94,60 | 97,40 | 97,85 | 97,50 | 97,70 | 97,90 | 97,91 | 97,41 |

| | | | | | | | | | |
|---|---|---|---|---|---|---|---|---|---|
| **absence de réacteur de finition | | | | | | | | | |

De façon surprenante, la mise en oeuvre du procédé permet d'augmenter selon l'invention la conversion globale d'isobutène avec une colonne catalytique contenant moins de zones réactionnelles dans les modes de réalisation des exemples 4 à 9 (5 zones réactionnelles au lieu de 9 zones réactionnelles). Sachant qu'une zone réactionnelle dans une colonne demande environ 3 mètres de hauteur, la hauteur de la colonne de distillation-réaction peut être diminuée d'environ 12 mètres, ce qui représente une diminution conséquente de l'investissement à réaliser.

Par ailleurs, la section réactionnelle supplémentaire, lorsqu'elle existe, est en dehors de la colonne de distillation et peut par conséquent fonctionner dans des conditions optimales et indépendantes de température et de pression permettant ainsi de maximiser la conversion d'isobutène. C'est ce que montre par exemple la comparaison des résultats obtenus dans le cas de la figure 7 comparés à ceux obtenus dans le cas représenté par la figure 6.

## Revendications

1. Dispositif de distillation et de réaction pour la fabrication d'un éther alkylique tertiaire par réaction d'au moins une oléfine avec monoalcool aliphatique, comprenant :
• un réacteur d'éthérification (R1) comportant un moyen d'alimentation,
• une section de distillation-réaction comprenant
- une colonne de distillation (F1), adaptée à séparer l'éther alkylique tertiaire au fond de ladite colonne,
- une colonne de distillation-réaction (F2), comprenant un lit de catalyseur d'éthérification,
- un réacteur complémentaire d'éthérification (R2), comportant un lit de catalyseur, le réacteur complémentaire comprend un conduit d'introduction et un conduit d'évacuation,
• un moyen de circulation d'un effluent dudit réacteur (R1) à ladite colonne de distillation (F1),
• un moyen de circulation d'un effluent de la tête de ladite colonne de distillation (F1) dans ladite colonne de distillation-réaction (F2),
• un moyen de prélèvement (9) de produit en fond de la colonne de distillation (F1),
ledit réacteur complémentaire (R2) est agencé selon l'une des dispositions suivantes :
a) ledit conduit d'introduction (16-17) et ledit conduit d'évacuation (18-19) sont reliés à ladite colonne de distillation (F1), et un moyen de circulation (7-8) conduit un effluent du fond de ladite colonne de distillation-réaction (F2) à la tête de ladite colonne de distillation (F1),
b) ledit conduit d'introduction (16-17) et ledit conduit d'évacuation (18-19) sont reliés à ladite colonne de distillation-réaction (F2), et un moyen de circulation (7-8) conduit un effluent du fond de ladite colonne de distillation-réaction (F2) à la tête de ladite colonne de distillation (F1),
c) ledit conduit d'introduction est relié au fond de ladite colonne de distillation-réaction et ledit conduit d'évacuation est relié à la tête de ladite colonne de distillation.

2. Dispositif selon la revendication 1 tel que ladite colonne de distillation-réaction (F2) comporte au moins un dispositif de reflux, ledit dispositif de reflux comprend au moins un moyen de prélèvement (2) situé en tête de ladite colonne de distillation-réaction (F2), alimentant au moins une zone de condensation (B1-E1), au moins un moyen de circulation (4-P1-5) d'une partie d'effluent de ladite zone de condensation vers la tête de ladite colonne de distillation-réaction (F2) et au moins un moyen de prélèvement (6) d'une autre partie d'effluent de la zone de condensation.

3. Dispositif selon l'une des revendications 1 et 2, et agencé selon la disposition a), dans lequel ledit conduit d'introduction (16-17) et ledit conduit d'évacuation (18-19) sont disposés à un même niveau de ladite colonne de distillation (F1).

4. Dispositif selon la revendication 3 tel que ledit niveau est situé entre l'alimentation de ladite colonne de distillation (F1) et la tête de ladite colonne de distillation (F1).

5. Dispositif selon l'une des revendications 1 et 2, et agencé selon la disposition b), dans lequel ledit conduit d'introduction (16-17) et ledit conduit d'évacuation (18-19) sont disposés à un même niveau de ladite colonne de distillation-réaction (F2).

6. Dispositif selon la revendication 5 dans lequel ledit niveau est situé entre le fond de ladite colonne de distillation-réaction (F2) et le bas du lit de catalyseur situé le plus bas dans ladite colonne de distillation-réaction (F2).

7. Dispositif selon l'une des revendications 1 à 6 dans lequel ledit conduit d'introduction du réacteur complémentaire comprend au moins un moyen de régulation de la température (E2).

8. Dispositif selon l'une des revendications 1 à 7 dans lequel ledit conduit d'évacuation du réacteur complémentaire comprend au moins un moyen de régulation de la température (E3).

9. Dispositif selon l'une des revendications 1 à 8 tel que ledit réacteur complémentaire (R2) comprend au moins un moyen d'alimentation de réactif.

10. Utilisation du dispositif selon l'une des revendications précédentes pour préparer d'au moins un éther alkylique tertiaire par réaction d'au moins un monoalcool aliphatique et d'au moins une oléfine, dans laquelle :
a) on introduit dans ledit réacteur au moins une oléfine et au moins un alcool aliphatique,
b) on prélève en fond de la colonne de distillation de l'éther alkylique tertiaire,
c) on prélève en tête de la colonne de distillation-réaction un effluent comprenant des hydrocarbures n'ayant pas réagi et de l'alcool aliphatique.

11. Utilisation selon la revendication 10 dans laquelle on introduit de l'alcool aliphatique supplémentaire dans ledit réacteur complémentaire.

## Patentansprüche

1. Destillations- und Reaktionsvorrichtung für die Herstellung eines tertiären Alkylethers durch Reaktion wenigstens eines Olifens mit einem aliphatischen Monoalkohol, umfassend:
• einen Veretherungsreaktor (R1) mit einem Speisemittel,
• einen Destillations-Reaktionsabschnitt, umfassend
- eine Destillationskolonne (F1), die geeignet ist, den tertiären Alkylether am Boden dieser Kolonne abzutrennen,
- eine Destillation-Reaktionskolonne (F2) ein Veretherungskatalysatorbett umfassend,
- einen komplementären Veretherungsreaktor (R2), der ein Katalysatorbett umfasst, wobei der komplementäre Reaktor eine Einführungsleitung und eine Abzugsleitung aufweist,
• ein Mittel, um einen Abstrom dieses Reaktors (R1) gegen diese Destillationskolonne (F1) in Zirkulation zu versetzen,
• ein Mittel, um einen Abstrom vom Kopf der Destillationskolonne (F1) in diese Destillations-Reaktionskolonne (F2) zu bewegen,
• ein Produktentnahmemittel (9) am Boden der Destillationskolonne (F1),
wobei dieser komplementäre Reaktor (R2) gemäß einer der folgenden Anordnungen ausgebildet ist:
a) die Einführungsleitung (16-17) und diese Abzugsleitung (18-19) sind mit dieser Destillationskolonne (F1) verbunden und ein Zirkulationsmittel (7-8) führt einen Abstrom vom Boden dieser Destillations-Reaktionskolonne (F2) zum Kopf dieser Destillationskolonne (F1),
b) diese Einführungsleitung (16-17) und diese Abzugsleitung (18-19) sind mit dieser Destillations-Reaktionskolonne (F2) verbunden und ein Zirkulationsmittel (7-8) führt einen Abstrom vom Boden dieser Destillatlons-Reaktionskolonne (F2) zum Kopf dieser Destillationskolonne (F1), und
c) diese Einführungsleitung ist mit dem Boden dieser Destillations-Reaktionskolonne verbunden und diese Abzugsleitung ist mit dem Kopf dieser Destillationskolonne verbunden.

2. Vorrichtung nach Anspruch 1, derart, dass diese Destillations-Reaktionskolonne (F2) wenigstens eine Rückstromeinrichtung umfasst, diese Rückstromeinrichtung wenigstens ein Entnahmemittel (2) umfasst, das am Kopf dieser Destillations-Reaktionskolonne (F2) angeordnet ist und wenigstens eine Kondensationszone (B1-E1) speist sowie wenigstens ein Zirkulationsmittel (4-P1-5) für einen Teil des Abstroms aus dieser Kondensationszone zum Kopf dieser Destillations-Reaktionskolonne (F2) und wenigstens ein Entnahmemittel (6) für einen anderen Teil des Abstroms aus der Kondensationszone aufweist.

3. Vorrichtung nach einem der Ansprüche 1 und 2 und ausgebildet gemäß Anordnung a), bei dem diese Einführungsleitung (16-17) und diese Abzugsleitung (18-19) auf ein und dem gleichen Niveau der Destillationskolonne (F1) angeordnet sind.

4. Vorrichtung nach Anspruch 3, derart, dass dieses Niveau zwischen der Speisung dieser Destillationskolonne (F1) und dem Kopf dieser Destillationskolonne (F1) angeordnet ist.

5. Vorrichtung nach einem der Ansprüche 1 und 5 und ausgebildet gemäß Anordnung b), bei der diese Einführungsleitung (16-17) und diese Abzugsleitung (18-19) auf ein und dem gleichen Niveau dieser Destillations-Reaktionskolonne (F2) angeordnet sind.

6. Vorrichtung nach Anspruch 5, bei dem dieses Niveau zwischen dem Boden dieser Destillations-Reaktionskolonne (F2) und dem unteren Bereich des Katalysatorbetts angeordnet ist, welches in dieser Destillations-Reaktionskolonne (F2) am tiefsten vorgesehen ist.

7. Vorrichtung nach einem der Ansprüche 1 bis 6, wobei diese Einführungsleitung des komplementären Reaktors wenigstens ein Temperatursteuermittel (E2) umfasst.

8. Vorrichtung nach einem der Ansprüche 1 bis 7, bei dem diese Abzugsleitung aus dem komplementären Reaktor wenigstens ein Temperatursteuermittel (E3) umfasst.

9. Vorrichtung nach einem der Ansprüche 1 bis 8, derart, dass dieser komplementäre Reaktor (R2) wenigstens ein Speisemittel für das Reaktionsmittel umfasst.

10. Verwendung der Vorrichtung nach einem der vorhergehenden Ansprüche zum Herstellen wenigstens eines tertiären Alkylethers durch Reaktion wenigstens eines aliphatischen Monoalkohols und wenigstens eines Olefins, bei der:
a) man in diesem Reaktor wenigstens ein Olefin und wenigstens einen aliphatischen Alkohol einführt,
b) man am Boden der Destillationskolonne tertiären Alkylether entnimmt,
c) man am Kopf der Destillations-Reaktionskolonne einen Abstrom entnimmt, der nicht umgesetzte Kohlenwasserstoffe und aliphatischen Alkohol umfasst.

11. Verwendung nach Anspruch 10, bei der man zusätzlichen aliphatischen Alkohol in diesem komplementären Reaktor einführt.

## Claims

1. A distillation and reaction device for preparing a tertiary alkyl ether by reaction of at least one olefin with an aliphatic monoalcohol, comprising:
• an etherification reactor (R1) comprising a supply means,
• a distillation-reaction section comprising:
- a distillation column (F1) suited to separate the tertiary alkyl ether at the bottom of said column,
- a distillation-reaction column (F2) comprising an etherification catalyst bed,
- a complementary etherification reactor (R2) comprising a catalyst bed, the complementary reactor including a supply line and a discharge line,
• a means intended for circulation of an effluent from said reactor (R1) to said distillation column (F1),
• a means intended for circulation of an effluent from the top of said distillation column (F1) to said distillation-reaction column (F2),
• a means (9) for drawing off product at the bottom of distillation column (F1),
said complementary reactor (R2) being laid out according to one of the following arrangements:
a) said supply line (16-17) and said discharge line (18-19) are connected to said distillation column (F1), and a circulation means (7-8) leads an effluent from the bottom of said distillation-reaction column (F2) to the top of said distillation column (F1),
b) said supply line (16-17) and said discharge line (18-19) are connected to said distillation-reaction column (F2), and a circulation means (7-8) leads an effluent from the bottom of said distillation-reaction column (F2) to the top of said distillation column (F1),
c) said supply line is connected to the bottom of said distillation-reaction column and said discharge line is connected to the top of said distillation column.

2. A device as claimed in claim 1, such that said distillation-reaction column (F2) comprises at least one reflux device, said reflux device comprising at least one draw-off means (2) arranged at the top of said distillation-reaction column (F2), feeding at least one condensation zone (B1-E1), at least one means (4-P1-5) intended for circulation of part of the effluent from said condensation zone to the top of said distillation-reaction column (F2) and at least one means (6) for drawing off another part of the effluent from the condensation zone.

3. A device as claimed in any one of claims 1 or 2 and laid out according to arrangement a), wherein said supply line (16-17) and said discharge line (18-19) are arranged at the same level in said distillation column (F1).

4. A device as claimed in claim 3, such that said level is located between the feed point of said distillation column (F1) and the top of said distillation column (F1).

5. A device as claimed in any one of claims 1 or 2 and laid out according to arrangement b), wherein said supply line (16-17) and said discharge line (18-19) are arranged at the same level of said distillation-reaction column (F2).

6. A device as claimed in claim 5, wherein said level is located between the bottom of said distillation-reaction column (F2) and the bottom of the lowest catalyst bed in said distillation-reaction column (F2).

7. A device as claimed in any one of claims 1 to 6, wherein said supply line of the complementary reactor comprises at least one temperature control means (E2).

8. A device as claimed in any one of claims 1 to 7, wherein said discharge line of the complementary reactor comprises at least one temperature control means (E3).

9. A device as claimed in any one of claims 1 to 8 such that said complementary reactor (R2) comprises at least one reactant feed means.

10. Use of the device as claimed in any one of the previous claims for preparing at least one tertiary alkyl ether by reaction of at least one aliphatic monoalcohol and at least one olefin, wherein:
a) at least one olefin and at least one aliphatic alcohol are fed into said reactor,
b) tertiary alkyl ether is drawn off at the bottom of the distillation column,
c) an effluent comprising unreacted hydrocarbons and aliphatic alcohol is drawn off at the top of the distillation-reaction column.

11. Use as claimed in claim 10, wherein additional aliphatic alcohol is fed into said complementary reactor.
